# EUROPEAN PATENT APPLICATION

(11) **EP 0 813 871 A1**
(43) Date of publication of application: **29.12.1997**
(21) Application number: 97109805.8
(22) Date of filing: 17.06.1997
(51) Int. Cl.: A61K 31/435

(54) **Berberine derivates for inhibiting production of HSP47**

(30) Priority: 18.06.1996 JP 177534/96
(71) Applicant: Kureha Chemical Industry Co., Ltd., Tokyo 103 (JP)
(72) Inventor: Kiyosuke, Yoichi, Tokyo 169 (JP); Tsuduki, Tomoko, Warabi-shi, Saitama 335 (JP); Shirakami, Toshiharu, Kodaira-shi, Tokyo 187 (JP); Morino, Masayoshi, Tokyo 179 (JP); Yoshikumi, Chikao, Kunitachi-shi, Tokyo 186 (JP)
(74) Representative: Cohausz & Florack

(57) **Abstract**

A pharmaceutical composition comprising a berberine derivative of the formula (I): 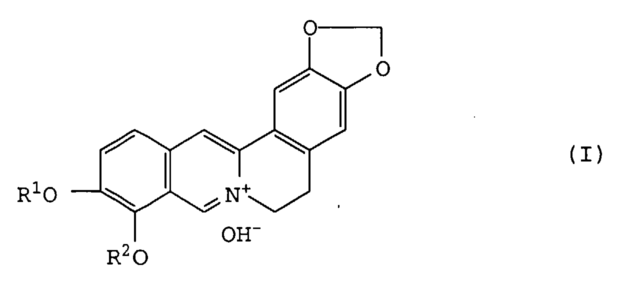 wherein R¹ and R² are independently an alkyl group of 1 to 3 carbon atoms, or a pharmaceutically acceptable salt thereof, as a substance inhibiting HSP47 production, and a pharmaceutically acceptable carrier, is disclosed. The substance inhibiting HSP47 production can effectively improve the physiological conditions of a patient suffering from diseases exhibiting a pathosis of an overproduction of the extracellular matrix, and efficiently treat such diseases. Further, the substance is useful for preventing or treating various diseases accompanied by an abnormal growth of vascularization.

## Description

### BACKGROUND OF THE INVENTION

### 1. Field of the Invention

The present invention relates to a pharmaceutical composition containing a berberine derivative or a pharmaceutically acceptable salt thereof as a substance inhibiting production of a heat shock protein having a molecular weight of 47 KDa (hereinafter referred to as HSP47).

In particular, the berberine derivative of the present invention can inhibit collagen synthesis in organs, to thereby improve physiological conditions of patients suffering from diseases exhibiting a pathosis of an overproduction of an extracellular matrix, for example, liver cirrhosis, interstitial lung disease, chronic renal failure (or diseases leading thereto), cardiac hypertrophy, postoperative scars, burn scars, keloid or hypertrophic scars remaining after a traffic accident or other accidents, scleroderma, arteriosclerosis, or rheumatoid arthritis, or to effectively treat said diseases.

### 2. Description of the Related Art

Diseases exhibiting a pathosis of an overproduction of an extracellular matrix, such as collagen, have now become a serious problem. The diseases exhibiting a pathosis of an overproduction of an extracellular matrix include, for example, liver cirrhosis, interstitial lung disease, chronic renal failure (or disease leading thereto), cardiac hypertrophy, postoperative scars, burn scars, keloid or hypertrophic scars remaining after a traffic accident or other accidents, scleroderma, arteriosclerosis, or rheumatoid arthritis.

The number of deaths by liver cirrhosis is estimated to have risen to about twenty thousand a year in Japan. Liver cirrhosis is a generic name of diseases in which the liver is hardened by a connective tissue growth. Liver cirrhosis is a diffuse hepatic fibrosis spread throughout the liver, and is considered to be the most serious of chronic liver diseases. More particularly, people with chronic hepatitis undergo a prolonged suffering from liver disorders, such as inflammation, which is accompanied by a considerable overproduction of an extracellular matrix, particularly the type I collagen, in the fibroblasts and/or Ito cells or the like, and thus a fibrosis of the liver occurs. If the fibrosis of the liver chronically increases, the normal regeneration of the liver becomes progressively inhibited. The fibroblasts and the extracellular matrices mainly composed of the type I collagen are substituted for the hepatocytes, and predominantly occupy the liver tissue, and thus a liver cirrhosis comprising many pseudolobules occurs. As the liver cirrhosis progresses, fibrous septums spread throughout the whole liver and cause hemostasis. The hemostasis partially causes a further degeneration of the liver parenchymal cells. As discussed above, this vicious cycle of the liver cirrhosis continues, and many collagen fibers are formed in the liver due to various causes, such as alcohol, virus, autoimmunity, or the like. Thereafter, a necrosis and dysfunction of the hepatocyte occur, and this leads to the death of the patient suffering from the liver cirrhosis. The type I collagen accounts for about 2% of all of the proteins in a normal liver, but for 10 to 30% in the liver cirrhosis.

The interstitial lung disease is a syndrome characterized by a chronic inflammation in a lower respiratory tract including not only alveoli and alveolar ducts, but also respiratory bronchioles and terminal bronchioles, such as alveolitis, and the fibrosis of the stroma and the fibrosis in the alveoli as a result thereof. The term "interstitial lung disease" includes, for example, interstitial pneumonia, diffuse interstitial lung disease, such as pulmonary fibrosis, idiopathic pulmonary fibrosis, permeability pulmonary edema, interstitial pneumonia associated with collagen vascular disease, sarcoidosis, or the like. In the interstitial lung disease, an overproduction and accumulation of the extracellular matrices are observed in the fibrotic tissues. In the fibrotic tissues of the interstitial lung disease, the type I and type III collagens are accumulated in a hypertrophic stroma to a remarkable extent. Particularly, the type III collagen is accumulated in the hypertrophic alveolar septa at an early stage of the fibrosis. The type I collagen is increased at a later stage and becomes a major collagen. The basement membrane is destroyed at the early stage, and an invasion of the collagen fibers into the alveolar space is observed.

The chronic renal failure denotes conditions wherein, as a result of the chronic nephritic syndrome, the renal function is severely damaged and is unable to maintain an endogenous homeostasis. From a pathological point of view, the progress of a chronic renal failure corresponds to the progress of the glomerular sclerosis and the fibrosis of the stroma. In the glomerulosclerosis, the hyperplasia of the extracellular matrix occurs mainly in the mesangial region. In the mesangial sclerosis, the components in the glomerular basement membrane, such as the type IV collagen, have a remarkable increase in comparison with a normal mesangial region, and the hyperplasia of the type I collagen, which is the component of the stroma, is observed in the regions corresponding to those of the scleroma. The chronically progressing glomerular sclerosis is mainly caused by the hyperplasia of the extracellular matrix. The diseases leading to a chronic renal failure include, for example, IgA nephropathy, focal glomerulosclerosis, memoranoproliferative nephritis, diabetic nephropathy, chronic interstitial nephritis, chronic glomerulonephritis, or the like.

A myocardial cell is highly differentiated, and thus cannot be proliferated by cell division. When a load is applied to a heart, each myocardial cell is hypertrophied to increase a contraction force and thereby maintain the cardiac function. It is known, however, that if such a load is further applied to a heart for a long time, various disorders including a cause of ischemia are accumulated, a compensation mechanism for a load fails, the contraction force is sharply reduced, the pump function of the heart ceases, and a heart failure occurs. In Japan, cardiac hypertrophy is a major cause of heart failures. Further, the formation of the cardiac hypertrophy not only becomes a main factor in the occurrence of heart failure, but also significantly raises a complication rate of ischemic heart diseases or severe ventricular arrhythmia, and thus leads to an independent defining of a vital prognosis. During the evolving of the cardiac hypertrophy, not only is each myocardial cell considerably hypertrophied, but also a fibrosis of the stroma is facilitated, to thus firmly bundle the hypertrophied myocardial cells, and the extracellular matrix, i.e., collagen, is increased. Further, if myocardial cells are lost due to myocarditis or myocardial ischemia, collagen is produced and substituted for the lost cells. When the fibrosis of the stroma progresses too far, the myocardium becomes hard, and thus the distension of the myocardium is inhibited. Further, the function of sarcoplasmic reticulum is lowered, and a relaxation of the diastole of the myocardium is inhibited.

Regarding the other diseases exhibiting a pathosis of an overproduction of an extracellular matrix, such as postoperative scars, burn scars, keloid or hypertrophic scars remaining after a traffic accident or other accidents, scleroderma, or arteriosclerosis, it is believed that an abnormal overproduction of collagen aggravates the fibrosis and then the screlosis of the tissue to thereby cause the diseases.

It has been reported that the basement membrane and the collagen synthesis therein play an important role in the vascularization [Maragoudakis, E., Sarmonika, M., and Panoutsacopoulous, M., "J. Pharmacol. Exp. Ther.", 244: 729, 1988; Ingber, D. E., Madri, J. A., and Folkman, J., "Endocrinology", 119: 1768, 1986]. The diseases known to be caused by the vascularization are, for example, diabetic retinopathy, retrolental fibroplasia, vascularization due to corneal transplantation, glaucoma, eye tumor, trachoma, psoriasis, pyogenic granuloma, hemangioma, angiofibroma, hypertrophic scars, granulation, rheumatoid arthritis, scleredema, atherosclerosis, or other tumors.

Although the diseases exhibiting a pathosis of an overproduction of the extracellular matrix, such as collagen, have become a severe problem, as mentioned above, an agent for inhibiting the production of the extracellular matrix, such as an agent for inhibiting the production of collagen, and satisfactory with regard to side effects, pharmacological effect, and so on, has not been developed. Heat shock proteins (HSPs) or stress proteins are a series of proteins expressed in a cell, when the cell is stimulated by a certain stress, such as heat, heavy metals, amino acid analogues, or a low concentration of oxygen. HSPs occur naturally and widely, and are produced in microorganisms, yeasts, plants, insects, and higher animals including humans.

HSPs include many types, and may be generally classified into four families, by molecular weight, i.e., an HSP90 family having an HSP of 90 kDa or 110kDa; an HSP70 family having an HSP of 70-73 kDa; an HSP60 family having an HSP of 57-68 kDa; and a small HSP family having an HSP of 20 kDa, 25-28 kDa or 47 kDa. In this specification, an HSP having a particular molecular weight is indicated by the term "HSP" followed by a "numerical figure". For example, an HSP having a molecular weight of 47 kDa is indicated by the term "HSP47".

As above, there exist many kinds of HSPs which are different not only in their molecular weights but also in structure, function or properties. Some of these proteins are synthesized constitutively in addition to the response to the stresses, and have been shown to play physiologically essential roles including a folding and unfolding of proteins, assembly of the subunits of proteins, and a membrane transport of proteins, under normal conditions. These functions of the heat shock protein are called a molecular chaperone.

HSP47, found by Nagata, et al. in 1986, is a basic protein (pI = 9.0) having a molecular weight of 47 kDa. Many reports show that, when the production of the HSP47 is increased, the production of collagen is increased ["J. Biol. Chem.", 261: 7531, 1986; "Eur. J. Biochem.", 206: 323, 1992; "J. Biol. Chem.", 265: 992, 1990; "J. Clin. Invest.", 94: 2481, 1994]. More particularly, it is believed that HSP47 serves as a molecular chaperone specific for collagen, which is presumably involved in the processing and/or the triple helix formation of procollagen in the endoplasmic reticulum, or transporting or secreting of procollagen from an endoplasmic reticulum to a Golgi apparatus. The increased production of HSP47 stimulates an accumulation of collagen molecules in the extracellular matrix. As mentioned above, the collagen-binding heat shock protein, HSP47, is closely associated with collagen, which is an extracellular matrix protein, from the viewpoint of production and function.

### SUMMARY OF THE INVENTION

The inventors of the present invention engaged in intensive studies into the provision of an agent for inhibiting the production of the extracellular matrix which can efficiently improve physiological conditions of patients suffering from the diseases exhibiting a pathosis of an overproduction of an extracellular matrix, for example, liver cirrhosis, interstitial lung disease, chronic renal failure (or disease leading thereto), cardiac hypertrophy, postoperative scars, burn scars, keloid or hypertrophic scars remaining after a traffic accident or other accidents, scleroderma, arteriosclerosis, or rheumatoid arthritis, and efficiently treat said diseases.

As explained above, a main lesion of the fibrosis, such as liver cirrhosis, interstitial lung disease, chronic renal failure (or diseases leading thereto), cardiac hypertrophy, postoperative scars, burn scars, keloid or hypertrophic scars remaining after a traffic accident or other accidents, scleroderma, arteriosclerosis, or rheumatoid arthritis is understood to be a pathosis whereby the extracellular matrix is remarkably increased in an organ. It is believed that the fibrosis accompanied with the diseases exhibiting a pathosis of an overproduction of the extracellular matrix, such as liver cirrhosis, interstitial lung disease, chronic renal failure (or diseases leading thereto), cardiac hypertrophy, postoperative scars, burn scars, keloid or hypertrophic scars remaining after a traffic accident or other accidents, scleroderma, arteriosclerosis, or rheumatoid arthritis, is caused by the increased biosynthesis of collagen or the reduced degradation of collagen. For example, a synthesis of the types I, III and IV collagens is activated in the fibrosis of the liver, and in particular, the activation of the synthesis of the type I collagen, which is a major component, is important.

Under these circumstances, the present inventors unexpectedly found that berberine contained in Phellodendri Cortex or Coptidis Rhizoma, a berberine derivative, or a pharmaceutically acceptable salt thereof can specifically inhibit the production of HSP47 in cells belonging to the tissue exhibiting a pathosis. More particularly, the present inventors found that the production of HSP47 in a cell, and the production of collagen in an organ, can be inhibited by administering the berberine derivative, or the pharmaceutically acceptable salt thereof, whereby diseases exhibiting a pathosis of an overproduction of the extracellular matrix, such as liver cirrhosis, interstitial lung disease, chronic renal failure (or diseases leading thereto), cardiac hypertrophy, postoperative scars, burn scars, keloid or hypertrophic scars remaining after a traffic accident or other accidents, scleroderma, arteriosclerosis, or rheumatoid arthritis can be treated. The present invention is based on these findings.

The object of the present invention is to provide an agent for inhibiting the production of HSP47 which is the collagen-specific molecular chaperone playing an important role in the processes of maturing and transporting collagen in a cell, and the agent can efficiently treat the diseases exhibiting a pathosis of an overproduction of the extracellular matrix, such as liver cirrhosis, interstitial lung disease, chronic renal failure (or diseases leading thereto), cardiac hypertrophy, postoperative scars, burn scars, keloid or hypertrophic scars remaining after a traffic accident or other accidents, scleroderma, arteriosclerosis, or rheumatoid arthritis.

Japanese Unexamined Patent Publication (Kokai) No. 2-69416 discloses an agent for improving a hepatopathy which contains berberine as an active ingredient. It further discloses that this agent effectively improved a survival rate of mice to which Propionibacterium acnes (P.acnes) was intraperitoneally administered, and to which lipopolysaccharide was intraperitoneally administered 7 days later. However, the mouse model used for the disease is reported to be a systemic model, because when sensitized with P. acnes it reacted with a small amount of lipopolysaccharide and the blood pressure became remarkably lower, and further, because not only the acute hepatopathy, such as infiltration of monocytes into hepatic lobes or necrosis of hepatocytes surrounding the hepatic lobes, but also lesions in the spleens and lungs were observed ("Japanese Pharmacology & Therapeutics", 22: Supplement, 161-165, 1994). The above Kokai Publication No. 2-69416 does not disclose or suggest the effect of berberine on the liver tissue, and thus, has little scientific ground to suggest that berberine improves hepatopathy. Further, it does not disclose or suggest that berberine functions to inhibit a production of HSP47, a production of collagen, an overproduction of an extracellular matrix, or a fibrosis of organs.

Further, Japanese Unexamined Patent Publication (Kokai) No. 4-193830 discloses an agent for improving nephritis, and this contains berberine as an active ingredient. It further discloses that the agent effectively improved anti-GEM (glomerular basement membrane) nephritis but does not disclose or suggest that berberine functions to inhibit a production of HSP47, a production of collagen, an overproduction of an extracellular matrix, or a fibrosis of organs.

The inventors of the present invention, however, found that the administration of berberine can inhibit a production of HSP47, a production of collagen in organs, and thus, inhibit a production of the extracellular matrix, to thereby prevent fibrosis and/or sclerosis of organs or tissues, effectively improve the pharmacological conditions of patients suffering from diseases exhibiting a pathosis of an overproduction of the extracellular matrix, such as liver cirrhosis, chronic renal failure or disease leading thereto, such as IgA nephropathy, focal glomerulosclerosis, membranoproliferative nephritis, diabetic nephropathy, chronic interstitial nephritis, or chronic glomerulonephritis, and effectively treat the diseases exhibiting a pathosis of an overproduction of the extracellular matrix, such as liver cirrhosis, chronic renal failure or diseases leading thereto.

The present invention is based on these findings.

Accordingly, the object of the present invention is to provide an agent for inhibiting the production of HSP47 which is the collagen-specific molecular chaperone playing an important role in the processes of maturing and transporting collagen in a cell, which agent can efficiently treat diseases exhibiting a pathosis of an overproduction of the extracellular matrix, such as liver cirrhosis, interstitial lung disease, chronic renal failure (or diseases leading thereto).

Other objects and advantages of the present invention will be apparent from the following description.

The present invention relates to a pharmaceutical composition, particularly an agent for inhibiting HSP47 production, comprising a berberine derivative of the formula (I): 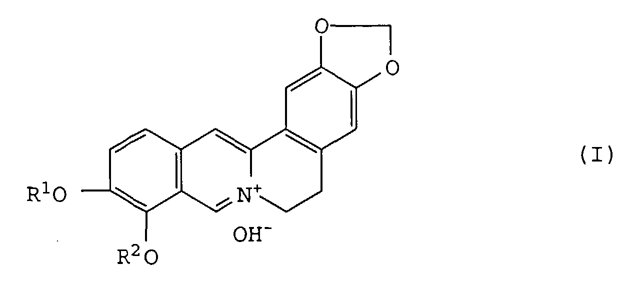 wherein R¹ and R² are independently an alkyl group of 1 to 3 carbon atoms, or a pharmaceutically acceptable salt thereof, as an active ingredient, and a pharmaceutically acceptable carrier.

Further, the present invention relates to a method for preventing or treating a disease exhibiting a pathosis of an overproduction of an extracellular matrix, comprising administering to a mammal in need thereof the berberine derivative of the formula (I) or a pharmaceutically acceptable salt thereof, in an effective amount for a prevention or treatment thereof.

Still further, the present invention relates to the use of the berberine derivative of the formula (I) or a pharmaceutically acceptable salt thereof, for preparing a pharmaceutical composition, particularly an agent for inhibiting HSP47 production.

### DESCRIPTION OF THE PREFERRED EMBODIMENTS

The present invention will be explained in detail hereinafter.

The pharmaceutical composition of the present invention contains the berberine derivative of the formula (I) or the pharmaceutically acceptable salt thereof as an active ingredient, i.e., a substance inhibiting HSP47 production. The alkyl group of 1 to 3 carbon atoms includes, for example, methyl, ethyl, n-propyl, or isopropyl group. In the compound of the formula (I), R¹ and R² are independently an alkyl group of 1 to 3 carbon atoms.

Of the berberine derivatives of the formula (I) which may be used as the substance inhibiting HSP47 production, according to the present invention, a berberine of the formula (II): 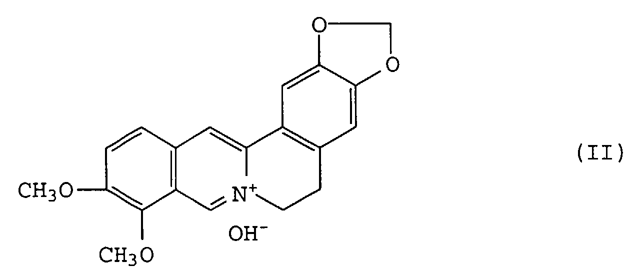 is preferable. The berberine is contained in a crude drug, for example, 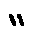 Phellodendri Cortex*"* or 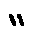 Coptidis Rhizoma*"*.

The pharmaceutically acceptable salts of the berberine derivative which may be used as the substance inhibiting HSP47 production according to the present invention include, for example, the pharmaceutically acceptable salts with organic or inorganic acids. As the salts, there may be mentioned, for example, hydrochlorides, sulfates, methanesulfonates, or p-toluenesulfonates, and further, salts with dicarboxylic acids, such as oxalic, malonic, succinic, maleic, or fumaric acid, or monocarboxylic acids, such as acetic, propionic, or butyric acid. The hydrochlorides, such as berberine chloride, i.e., the compound of the formula (III): 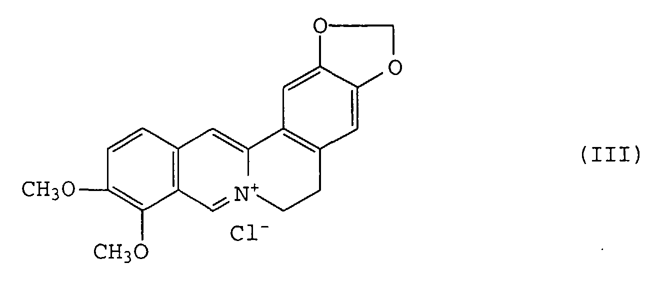 is particularly preferable.

The berberine derivative or the pharmaceutically acceptable salt thereof which may be used as the substance inhibiting HSP47 production in the present invention may be prepared by chemical synthesis or extraction of a naturally-occurring material followed by purification of the extract. Alternatively, a commercially available berberine derivative or the pharmaceutically acceptable salt thereof may be used. When the berberine derivative or the pharmaceutically acceptable salt thereof which may be used as the substance inhibiting HSP47 production according to the present invention is extracted from a natural material, the whole plant containing the berberine derivatives or the pharmaceutically acceptable salts thereof, or a part of the plant, such as whole grasses, leaf, root, rhizome, stem, root bark or flowers, is extracted without further treatment, or after a simple treatment such as drying, cutting, scalding, heating by steam or pulverizing, to prepare crude drugs. The extraction conditions are not limited as long as they are the conditions generally used for plant extraction. The plant containing berberine is not particularly limited and for example, 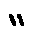 Phellodendron amurense Ruprecht*"* or 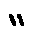 Coptis japonica Makino*"* may be used.

The berberine or the pharmaceutically acceptable salt thereof extracted from a crude drug as the substance inhibiting HSP47 production is not particularly limited to, but is preferably extracted from 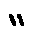 Phellodendri Cortex*"* or 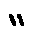 Coptidis Rhizoma*"*. 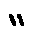 Phellodendri Cortex; Phellodendron Bark*"* is a bark prepared from Phellodendron amurense Ruprecht or 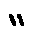 Rutaceae*"* by removing periderm. Any parts of the Phellodendri Cortex may be used alone or in a combination thereof. 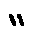 Coptidis Rhizoma; Coptis Rhizome*"* is a rhizome prepared from Coptis japonica Makino or 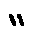 Ranunculaceae*"* by removing almost all of the root. Any parts of the Coptidis Rhizoma may be used alone or in a combination thereof.

A Phellodendri Cortex or Coptidis Rhizoma extract which may be used as the substance inhibiting HSP47 production in the present invention is not limited, as long as it contains the above-mentioned berberine or the pharmaceutically acceptable salt thereof. Thus, a crude Phellodendri Cortex or Coptidis Rhizoma extract may be used as the substance inhibiting HSP47 production according to the present invention. Phellodendri Cortex or Coptidis Rhizoma is extracted with, for example, cold water, warm water, or hot water, or an organic solvent, to prepare the Phellodendri Cortex or Coptidis Rhizoma extract which may be used as the substance inhibiting HSP47 production according to the present invention. As the organic solvent, there may be mentioned, for example, alcohols of 1 to 6 carbon atoms (such as methanol, ethanol, n-propanol, isopropanol, or butanol), ester (such as methyl acetate, ethyl acetate, propyl acetate, or butyl acetate), ketone (such as acetone, or methyl isobutyl ketone), ether, petroleum ether, n-hexane, cyclohexane, toluene, benzene, organohalogen compound (such as carbon tetrachloride, dichloromethane, or chloroform), pyridine, glycol (such as propylene glycol, or butylene glycol), polyethylene glycol, or acetonitrile. The organic solvents can be used alone or as a mixture of a suitable combination thereof at certain proportions. Further, anhydrous or aqueous organic solvents may be used. Preferably, methanol is used.

As the extraction with water or the organic solvent, an ordinary extraction method for obtaining crude drugs may be used. For example, it is preferable to add 3 to 300 parts by weight of water or organic solvent to 1 part by weight of (dried) Phellodendri Cortex or Coptidis Rhizoma, and then, heat the whole under reflux at a temperature below the boiling point, to carry out an ultrasonic extraction at an ordinary temperature, or to carry out a cold extraction, with stirring. The extraction procedure may be carried out generally for 5 minutes to 7 days, preferably for 10 minutes to 60 hours. If necessary an auxiliary means, such as stirring, may be used to shorten the extraction time. It is more preferable to defat the crude drugs with, for example, ether or benzene, before the extraction.

The product obtained by extraction with water or the organic solvent may be treated by a suitable process, such as filtration or centrifugation, to remove impurities and obtain a crude extract. The resulting crude extract may be used, without further purification, when the extracted and fractionated berberine derivative or the pharmaceutically acceptable salt thereof obtained from the naturally occurring material is used as the substance inhibiting HSP47 production according to the present invention. In addition to the products obtained by an extraction with water or organic solvent, the extract products obtained by treating the above crude extract further with various organic solvents or adsorbents also may be used as the substance inhibiting HSP47 production according to the present invention. The Phellodendri Cortex or Coptidis Rhizoma extract which is the crude extract or the various purified extract products may be used in the form of a liquid extract without any treatment other than extraction, a concentrated extract obtained by evaporating the solvents, a pulverized product obtained by evaporation of the solvents and drying, a purified product obtained by crystallization, a viscous product, or a diluted liquid thereof.

The resulting Phellodendri Cortex or Coptidis Rhizoma extracts retain berberine contained in Phellodendri Cortex or Coptidis Rhizoma, or the pharmaceutically acceptable salt thereof, and impurities from the starting Phellodendri Cortex or Coptidis Rhizoma.

It is possible to administer the berberine derivative or the pharmaceutically acceptable salt thereof, or the extract from the plant containing the berberine derivative or the pharmaceutically acceptable salt thereof, such as the extract from the crude drug containing the berberine derivative or the pharmaceutically acceptable salt thereof (particularly, the Phellodendri Cortex or Coptidis Rhizoma extract) according to the present invention, optionally together with preferably a pharmaceutically or veterinarily acceptable ordinary carrier, to an animal, preferably a mammal, particularly humans. The formulation is not limited to but may be, for example, oral medicines, such as powders, fine subtilaes, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants, or the like, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate. For example, the capsule may be prepared by mixing and filling 1 part by weight of berberine hydrochloride and 99 parts by weight of lactose.

As the parenteral administration, for example, an injection such as a subcutaneous or intravenous injection, or the per rectal administration may be used. This administration by injection is preferable.

When the injections are prepared, for example, water-soluble solvents, such as physiological saline or Ringer's solution, water-insoluble solvents, such as plant oil or fatty acid ester, tonicity agents, such as glucose or sodium chloride, solubilizing agents, stabilizing agents, antiseptics, suspending agents, emulsifying agents or like may be optionally used, in addition to the berberine derivative or the pharmaceutically acceptable salt thereof, or the extract from the plant containing the berberine derivative or the pharmaceutically acceptable salt thereof, such as the extract from the crude drug containing the berberine derivative or the pharmaceutically acceptable salt thereof (particularly, the Phellodendri Cortex or Coptidis Rhizoma extract), as the substance inhibiting HSP47 production according to the present invention.

The pharmaceutical composition may be administered in the form of a sustained release preparation using sustained release polymers. For example, the pharmaceutical composition of the present invention may be incorporated into a pellet made of ethylenevinyl acetate polymers, and the pellet may be implanted in a tissue to be treated.

The amount of the berberine derivative or the pharmaceutically acceptable salt thereof contained in the pharmaceutical composition of the present invention is not limited, in that the pharmaceutical composition may contain 0.01 to 99%, preferably 0.1 to 80% by weight of the berberine derivative or the pharmaceutically acceptable salt thereof.

The pharmaceutical composition of the present invention containing the extract from the plant containing the berberine derivative or the pharmaceutically acceptable salt thereof, such as the extract from the crude drug containing the berberine derivative or the pharmaceutically acceptable salt thereof (particularly, the Phellodendri Cortex or Coptidis Rhizoma extract), as an active ingredient may be prepared by appropriately adjusting the amount of the berberine derivative or the pharmaceutically acceptable salt thereof contained therein to the above range. When the pharmaceutical composition of the present invention containing the above extract is prepared as a pharmaceutical for oral administration, it is preferable to use a pharmaceutically acceptable carrier.

The dose of the pharmaceutical composition of the present invention varies with the kind of disease, the age, sex, body weight, symptoms, method of administration, or the like, but the berberine derivative may be orally or parenterally administered at a dosage of about 1 mg to 10 g a day for an adult, usually divided into one to four dosages.

The composition containing the berberine derivative of the formula (I) or the salt thereof as the substance inhibiting HSP47 production may be used not only for the pharmaceutical application but also for various applications, for example, in the form of food such as a functional food or health food.

The berberine derivative or the pharmaceutically acceptable salt thereof contained in the present pharmaceutical composition has a function of being able to specifically inhibit the production of HSP47 in cells. Therefore, when the berberine derivative or the pharmaceutically acceptable salt thereof according to the present invention are administered, the biosynthesis of HSP47 in cells is specifically inhibited, and thus, the biosynthesis of collagen is specifically inhibited. As a result, the production of the extracellular matrix is inhibited. Accordingly, the berberine derivative or the pharmaceutically acceptable salt thereof according to the present invention may be used for treating or preventing diseases exhibiting a pathosis of an overproduction of an extracellular matrix accompanied by a collagen increase, for example, liver cirrhosis, interstitial lung disease, chronic renal failure (or disease leading thereto), cardiac hypertrophy, postoperative scars, burn scars, keloid or hypertrophic scars remaining after a traffic accident or other accidents, scleroderma, arteriosclerosis, or rheumatoid arthritis. Namely, the pharmaceutical composition of the present invention inhibits the production of HSP47, which is a molecular chaperone specific to collagen, and thus inhibits the production of collagen.

Further, it has been reported that the basement membrane and the collagen synthesis therein play an important role in the vascularization, as mentioned above. Therefore, the pharmaceutical composition of the present invention is useful for preventing and treating many diseases caused by an abnormal growth of the vascularization, and can be used for the above-mentioned diseases, for example, diabetic retinopathy, retrolental fibroplasia, vascularization due to corneal transplantation, glaucoma, eye tumor, trachoma, psoriasis, pyogenic granuloma, hemangioma, angiofibroma, hypertrophic scars, granulation, rheumatoid arthritis, scleredema, atherosclerosis, or other tumors. Furthermore, it has been reported that the interstitial stroma having a basic structure of the type I collagen and fibronectin serves, in cancer metastasis, as a guide for the released cancer cells to enter the vessels in the vicinity [ 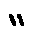 Biotherapy*"*, 7(8): 1181, 1993]. Therefore, cancer metastasis may be inhibited by administering the pharmaceutical composition of the present invention.

### Examples

The present invention will now be further illustrated by, but is by no means limited to, the following Examples.

### Example 1: Preparation of anti-HSP47 polyclonal antibody

### (1) Preparation of anti-HSP47 polyclonal antibody

A peptide consisting of 15 amino acids corresponding to the amino acid sequence from the second to 16th amino acids in the N-terminus of human HSP47 [hereinafter referred to as human HSP47 peptide (2-16)] was synthesized by an automatic peptide-synthesizer (PSSM-8 System; Shimadzu Corp., Japan). A sensitized antigen was prepared by binding the peptide with lactoglobulin, using succinimidyl 4-(p-maleimidophenyl)butyrate (SMPB) as a crosslinking agent, by an ordinary method ("Biochemistry", 18:690, 1979).

Then, 0.2 ml of phosphate buffered physiological saline [composition: KC1 = 0.2 g/l, KH₂PO₄ = 0.2 g/l, NaCl = 8 g/1, Na₂HPO₄ (anhydrous) = 1.15 g/l: hereinafter referred to as PBS(-): COSMO BIO CO. Catalogue No. 320-01] containing 150 µg of the sensitized antigen, and the same amount of Freund's complete adjuvant (Iatron, Catalogue No. RM606-1) were mixed, and 0.2 ml of the resulting liquid mixture was subcutaneously administered to a Lou rat (6-week old, female, CLEA JAPAN, INC.) for immunization. The same procedures were repeated for the second and third immunizations, and the immunizations using an adjuvant (Hunter's TiterMax; CytRx Corporation, Ga, USA) were repeated six times.

Blood was collected from the sensitized animal, and a serum was separated by an ordinary method. The antibody titer was determined by an enzyme immunoassay (ELISA) and Western blot technique.

### (2) Evaluation of properties of anti-HSP47 polyclonal antibody by enzyme immunoassay (ELISA)

The human HSP47 peptide (2-16) was dissolved in PBS(-) to obtain the peptide solution (10 µg/ml). The peptide solution was poured into wells of a rigid assay plate (Falcon; Catalogue No. 3910) at 50 µl/well, and 50 µl of only PBS(-) was poured into the well at the edge of the plate. The plate was allowed to stand at 4 °C overnight in a wet condition. After the peptide solution was discarded, the wells were washed with PBS(-), and 100 µl of PBS(-) containing 1% bovine serum albumin (hereinafter referred to as BSA) was poured into each well. The plate was allowed to stand at room temperature for 1 hour. After the wells were washed three times with PBS(-), 50 µl of the Lou rat serum obtained in the above (1) was added to each well. The plate was allowed to stand at room temperature for 1 hour. After the wells were washed three times with PBS(-), 50 µl of peroxidase-labeled anti-rat IgG was poured as a second antibody to each well. The plate was allowed to stand at room temperature for 1 hour.

After the wells were washed twice with PBS(-), 100 µl of a substrate solution prepared by dissolving one tablet (10 mg) of o-phenylene diamine (OPD) (Sigma, Catalogue No. P8287) in 10 ml of 0.1 M citrate buffer (pH 4.5) containing 4 µl of hydrogen peroxide solution was added dropwise to each well. The plate was allowed to stand at room temperature for 30 minutes while shielded from light. The absorption at 492 nm for each well was measured by a microplate reader (Toso, MPR-A4i type).

The sera having a high antibody titer were used as the anti-HSP47 polyclonal antibody in the following Examples.

### (3) Evaluation of properties of anti-HSP47 polyclonal antibody by Western blot technique

Sodium dodecyl sulfate (SDS)-polyacrylamide gel electrophoresis of the HeLa cell lysate was carried out, using Laemmli's buffer (Laemmli, N. K., "Nature", 283: pp.249-256, 1970), as follows:

A concentrated gel was prepared by mixing 6.1 ml of distilled water, 2.5 ml of 0.5 M Tris (Bio-Rad, Catalogue No. 161-0716)-HCl (pH 6.8), 100 µl of 10% SDS (Bio-Rad, Catalogue No. 161-0301), and 1.3 ml of 30% acrylamide (Bio-Rad, Catalogue No. 161-0101)/N,N'-methylene bisacrylamide (Bio-Rad, Catalogue No. 161-0201), deaerating for 15 minutes, and adding 50 µl of 10% ammonium persulfate (Bio-Rad, Catalogue No. 161-0700) and 10 µl of N,N,N',N'-tetramethylethylene-diamine (hereinafter, referred to as TEMED) (Bio-Rad, Catalogue No. 161-0800).

A separating gel was prepared by slowly mixing 4.045 ml of distilled water, 2.5 ml of 1.5 M Tris-HCl (pH 8.8), 100 µl of 10% SDS, and 3.3 ml of 30% acrylamide/N,N'-methylenebisacrylamide, deaerating for 15 minutes with an aspirator, and adding 50 µl of 10% ammonium persulfate, and 5 µl of TEMED.

An electrophoresis buffer was prepared by adding 9.0 g of Tris, 43.2 g of glycine (Bio-Rad, Catalogue No. 161-0717) and 3.0 g of SDS to distilled water to obtain 600 ml of a solution, and diluting the solution with distilled water to 5 fold.

A sample buffer was prepared by mixing 2 ml of distilled water, 500 µl of 2 M Tris-HCl (pH 6.8), 0.32 g of SDS, 800 µl of β-mercaptoethanol, and 400 µl of 0.05% (w/v) bromophenol blue (Bio-Rad, Catalogue No. 161-0404).

The HeLa cells were cultured under 5% CO₂ at 37 °C in an MEM medium containing 10% heat inactivated fetal bovine serum (hereinafter referred to as FBS), and the lysate was prepared. SDS-polyacrylamide gel electrophoresis of the HeLa cell lysate was carried out, and the gel was brought into close contact with a 0.45 µm nitrocellulose membrane (Schleicher & Schuell, Catalogue No. 401196). Blotting was carried out at room temperature and 100 V for 3 hours, using a protein-transferring apparatus (Trans-Blot Electrophoretic Transfer Cell: Bio-Rad). The blotting buffer used was prepared by adding methyl alcohol to Tris-glycine buffer (pH 8.5) consisting of 0.025 M Tris and 0.192 M glycine (Tris Gly Running and Blotting Buffer; Enprotech, Mass., USA; Catalogue No. SA100034), so that the concentration of methyl alcohol became 20%.

After blotting, the nitrocellulose membrane was dipped for 30 minutes at room temperature in a PBS(-) solution containing 5% skimmed milk (Snow Brand Milk) to perform blocking. After blocking, the first antibody reaction was carried out, using a screener blotter (Sanplatec) and the Lou rat serum obtained in the above (1) as the first antibody. The first antibody reaction was carried out in PBS(-) containing 2% skimmed milk (Snow Brand Milk) at room temperature for 120 minutes, using 200 µl of a 10-fold dilution of the Lou rat serum. After the first antibody reaction, the nitrocellulose membrane was washed, using a slow rocking shaker, by shaking twice for 5 minutes in PBS(-), four times for 15 minutes in a PBS(-) solution containing 0.1% Tween 20 (Bio-Rad, Catalogue No. 170-6531), and further, twice for 5 minutes in PBS(-).

After washing, the second antibody reaction was carried out for 2 hours, using 5 ml of a solution prepared by diluting a peroxidase-labeled goat anti-rat IgG antibody (Southern Biotechnology, Catalogue No. 3030-05) 5000-fold with a PBS(-) solution containing 2% skimmed milk. After the reaction, the nitrocellulose membrane was washed with a PBS(-) solution and a PBS(-) solution containing 0.1% Tween 20, under the same conditions as those used in the washing after the first reaction.

After the excess PBS(-) solution was removed, a Western blotting detection reagent (ECL Western blotting detection reagent; Amersham, Catalogue No. RPN2106) was sprinkled over the nitrocellulose membrane, and the membrane was allowed to stand at room temperature for 1 minute. After the excess detection reagent was removed, the nitrocellulose membrane was enclosed with a wrapping film. The reaction surface was placed in contact with an X-ray film (Kodak X-OMAT, AR; Catalogue No. 165 1454) and exposed. After the film was developed, a band around a molecular weight of 47 kDa, i.e., the molecular weight of HSP47, was detected to evaluate the reactivity of the anti-HSP47 polyclonal antibody.

The sera whose antibody titer was observed to be high were used as the anti-HSP47 polyclonal antibody in the following Example.

### Example 2: Inhibition of HSP production in human cancer cell lines

### (1) Culture of human cancer cell lines

The following various human cancer cell lines were cultured under 5% CO₂, at 37 °C, except for the heat shock treatment period. The lung cancer cell line H69 (ATCC HTB 119), the stomach cancer cell line KATO III (ATCC HTB 103), the colon cancer cell line COLO 205 (ATCC CCL 222), and the prostate cancer cell line DU145 (ATCC HTB 81) were cultured in an RPMI1640 medium containing 10% inactivated FBS.

The breast cancer cell line MCF7 (ATCC HTB 22) was cultured in an RPMI1640 medium containing 10⁻⁸ M β-estradiol and 10% inactivated FBS.

The kidney cancer cell line ACHN (ATCC CRL 1611) was cultured in an MEM medium containing 10% inactivated FBS.

### (2) Treatment with berberine hydrochloride and heat shock treatment

The berberine hydrochloride of the formula (III) (the final concentration = 20 µM: Matsuura Yakugyo) was added to each medium of the above human cancer cell lines 2 days after the inoculation. After the berberine hydrochloride treatment for 24 hours, a heat shock was applied at 45 °C for 15 minutes, and the cells were then cultured at 37 °C overnight. The control tests were carried out by performing the above procedures except that the berberine hydrochloride was not added.

### (3) Determination of amount of HSP produced in human cancer cell lines

The cells treated in the above (2) were homogenized, and the amounts of HSP47 produced were measured by the Western blotting method.

The cells treated in the above (2) were washed with PBS(-), and 1 ml of lysis buffer [1.0% NP-40, 0.15 M sodium chloride, 50 mM Tris-HCl (pH 8.0), 5 mM-EDTA, 2 mM-N-ethylmaleimide, 2 mM phenylmethylsulfonyl fluoride, 2 µg/ml leupeptin, and 2 µg/ml pepstatin] was added. The whole was allowed to stand for 20 minutes on ice, and then centrifuged at 4 °C and 12000 rpm for 20 minutes. The resulting supernatant (10 µl) was added to 790 µl of PBS(-), and then 200 µl of a protein assay dye reagent solution (Dye Reagent Concentrate: Bio-Rad Laboratories, Catalogue No. 500-0006) was added. The whole was allowed to stand at room temperature for 5 minutes, and the absorbance at 595 nm was measured to determine the amounts of proteins.

For the SDS-polyacrylamide gel electrophoresis (SDS-PAGE) of the samples, the protein concentration of which were measured, aliquots containing the same amount of protein were loaded onto the gel. The SDS-PAGE was performed according to the Laemmli method. After the electrophoresis, the blotting and then blocking were carried out in accordance with the methods described in Example 1. More particularly, the gel was brought into contact with a 0.45 µm nitrocellulose membrane (Schleicher & Schuell, Catalogue No. 401196) at room temperature and 100 V, using a protein-transferring apparatus (Trans-Blot Electrophoretic Transfer Cell: Bio-Rad), and blotting was carried out for 3 hours. The blotting buffer used was same as that used in Example 1 (3). After blotting, the nitrocellulose membrane was incubated in a 10% skimmed milk-PBS(-) solution at room temperature for 30 minutes, to block non-specific bindings.

After blocking, the first antibody reaction was carried out on the nitrocellulose membrane, using a screener blotter (Sanplatec), and the anti-human-HSP47 rat polyclonal antibody prepared in Example 1. Thereafter, the nitrocellulose membrane was washed with two fresh PBS(-) solutions for 5 minutes, using a slow rocking shaker, and then with four fresh PBS(-)-0.1% Tween 20 (Bio-Rad, Catalogue No. 170-6531) solutions for 15 minutes, and finally, with two fresh PBS(-) solutions for 5 minutes.

After washing, the second antibody reaction was carried out for 2 hours, using 5 ml of a solution prepared by diluting a peroxidase-labeled goat anti-rat IgG antibody (Southern Biotechnology, Catalogue No. 3030-05) 5000-fold with a PBS(-) solution containing 2% skimmed milk. After the reaction, the nitrocellulose membrane was washed with two fresh PBS(-) solutions for 5 minutes, and with five fresh PBS(-)-0.1% Tween 20 solutions for 15 minutes, using a slow rocking shaker, and finally, with two fresh PBS(-) solutions for 5 minutes. After the excess PBS(-) solution was removed, a Western blotting detection reagent (ECL Western blotting detection reagent; Amersham, Catalogue No. RPN2106) was sprinkled over the nitrocellulose membrane, and the membrane was incubated for 1 minute. After the excess detection reagent was removed, the nitrocellulose membrane was enclosed with a wrapping film. The reaction surface was placed in contact with an X-ray film (Kodak X-OMAT, AR; Catalogue No. 165 1454) and exposed. After the film was developed, the presence of HSP47 was detected. The results are shown in Table 1. In Table 1, the symbol "↓" means that the amounts of HSP47 produced were reduced by the berberine hydrochloride-treatment in comparison with the control tests.

**Table 1**

| cancer | cancer cell line | change in amount of HSP47 produced |
|---|---|---|
| lung | H69 | ↓ |
| stomach | KATO III | ↓ |
| colon | COLO 205 | ↓ |
| breast | MCF7 | ↓ |
| prostate | DU145 | ↓ |
| kidney | ACHN | ↓ |

In control tests, i.e., in the case of cell lines which were not treated with the berberine hydrochloride, a single band of a molecular weight of approximately 47 kDa was detected in the lung cancer cell line H69, the stomach cancer cell line KATO III, the colon cancer cell line COLO 205, the breast cancer cell line MCF7, prostate cancer cell line DU145, and the kidney cancer cell line ACHN. The molecular weight was determined by molecular weight markers (bovine carbonic anhydrase, hen egg white ovalbumin, and bovine serum albumin). In the case of cell lines treated with the berberine hydrochloride, no band of the molecular weight of approximately 47 kDa was detected in the lung cancer cell line H69, the stomach cancer cell line KATO III, the colon cancer cell line COLO 205, the breast cancer cell line MCF7, and prostate cancer cell line DU145. In the kidney cancer cell line ACHN, the detected band of the molecular weight of approximately 47 kDa was faint, in comparison with the control tests. Therefore, it can be concluded that the berberine hydrochloride has an activity that will inhibit the HSP47 production, and is useful as a substance inhibiting HSP47 production. Further, the above results show that the berberine hydrochloride inhibits an overproduction of the extracellular matrix.

As explained above, the pharmaceutical composition of the present invention contains the substance inhibiting HSP47 production, and therefore, has an activity that will lessen the collagen overproduction observed in cells suffering from diseases exhibiting a pathosis of an overproduction of the extracellular matrix, for example, liver cirrhosis, interstitial lung disease, chronic renal failure (or disease leading thereto), cardiac hypertrophy, postoperative scars, burn scars, keloid or hypertrophic scars remaining after a traffic accident or other accidents, scleroderma, arteriosclerosis, or rheumatoid arthritis. Accordingly, the administration of the pharmaceutical composition of the present invention can inhibit the fibrosis and the sclerosis of organs or tissues, efficiently improve the physiological conditions of a patient suffering from such diseases, and efficiently treat such diseases.

Further, the pharmaceutical composition of the present invention is useful for preventing or treating various diseases accompanied by an abnormal growth of the vascularization.

Furthermore, it is known that the interstitial stroma having a basic structure of the type I collagen and fibronectin serves, in cancer metastasis, as a guide for the released cancer cells to enter vessels in the vicinity. Therefore, cancer metastasis may be inhibited by administering the pharmaceutical composition of the present invention.

Although the present invention has been described with reference to specific embodiments, various changes and modifications obvious to those skilled in the art are deemed to be within the spirit, scope, and concept of the invention.

## Claims

1. A pharmaceutical composition comprising a berberine derivative of the formula (I): 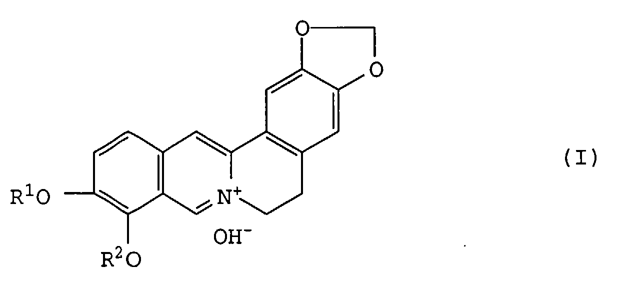 wherein R¹ and R² are independently an alkyl group of 1 to 3 carbon atoms, or a pharmaceutically acceptable salt thereof, as a substance inhibiting HSP47 production, and a pharmaceutically acceptable carrier.

2. The composition according to claim 1, wherein the berberine derivative is a compound of the formula (II):

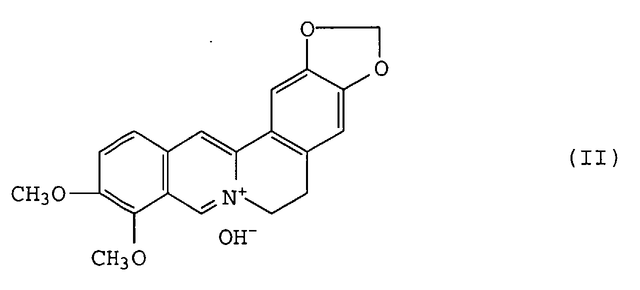

3. The composition according to claim 1, wherein the berberine derivative is a compound of the formula (III):

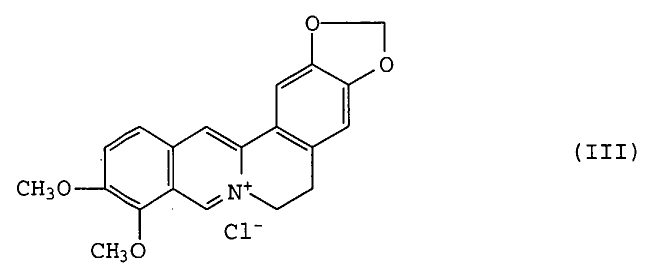

4. A pharmaceutical composition comprising an extract from a plant containing a berberine derivative of the formula (I): 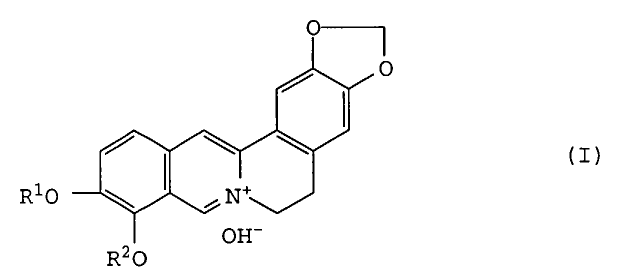 wherein R¹ and R² are independently an alkyl group of 1 to 3 carbon atoms, or a pharmaceutically acceptable salt thereof, as a substance inhibiting HSP47 production, and a pharmaceutically acceptable carrier.

5. A pharmaceutical composition comprising an extract from the group consisting of a Phellodendri Cortex extract and a Coptidis Rhizoma extract, as the substance inhibiting HSP47 production, and a pharmaceutically acceptable carrier.

6. Use of the berberine derivative of the formula (I) according to claim 1, or a pharmaceutically acceptable salt thereof, for the preparation of a pharmaceutical composition.

7. A food composition comprising the berberine derivative of the formula (I) according to claim 1.
